# EUROPÄISCHE PATENTSCHRIFT

(11) **EP 1 701 796 B1**
(45) Veröffentlichungstag und Bekanntmachung des Hinweises auf die Patenterteilung: **16.06.2010**
(21) Anmeldenummer: 05700298.2
(22) Anmeldetag: 04.01.2005
(51) Int. Cl.: B03C 1/28, G01N 33/543

(54) **TRENNEN UND REINIGEN EINER SUSPENSION MIT MAGNETISCHEN MIKROPARTIKELN**
SEPARATION AND CLEANING OF A SUSPENSION COMPRISING MAGNETIC MICROPARTICLES
SEPARATION ET PURIFICATION D'UNE SUSPENSION A L'AIDE DE MICROPARTICULES MAGNETIQUES

(30) Priorität: 08.01.2004 CH 242004
(43) Veröffentlichungstag der Anmeldung: 20.09.2006
(73) Patentinhaber: Qiagen Instruments AG, 8634 Hombrechtikon (CH)
(72) Erfinder: LUTZE, Konstantin, 8708 Männedorf (CH)
(74) Vertreter: Breiter, Heinz
(86) Internationale Anmeldenummer: PCT/CH2005/000001
(87) Internationale Veröffentlichungsnummer: WO 2005/065831

(56) Entgegenhaltungen:
- EP-A- 0 479 448
- EP-A- 0 806 665
- DE-A1- 3 926 462

## Beschreibung

Die Erfindung bezieht sich auf eine Vorrichtung zum automatischen Trennen der festen und flüssigen Phase einer Suspension und zum Reinigen von mit organischen, insbesondere molekularbiologischen oder biochemischen Substanzen beladenen, magnetischen Mikropartikeln, welche Vorrichtung eine Prozessarea mit getaktet wandernden Einrichtungen für den Transport der magnetischen Mikropartikel in x-Richtung umfasst. Weiter betrifft die Erfindung Proben- und Reagenzbehälter zum Einsatz in der Vorrichtung und ein Verfahren zum automatischen Trennen und Reinigen in der Vorrichtung.

Das Untersuchen und/oder Analysieren von organischen, insbesondere molekularbiologischen Substanzen durch Bestimmen ihrer chemischen oder physikalischen Eigenschaften mit spezifisch entwickelten Methoden gewinnt laufend an Bedeutung. Für die chemische Analyse von molekularbiologischen Stoffen, beispielsweise Blut oder Urin ist die Verwendung eines inerten Trägers in Form von Mikropartikeln vorteilhaft. Falls diese Mikropartikel aus einem magnetischen oder magnetisierbaren Werkstoff bestehen, einen solchen Werkstoff enthalten oder mit einem solchen überzogen sind, kann die feste Phase mit einem Magnetfeld aus einer Suspension abgetrennt und durch nachfolgende Reinigungs- bzw. Waschprozesse mit einem sehr hohen Sauberkeitsgrad isoliert werden. Nichtmagnetische Mikropartikel werden sedimentiert, abgesaugt oder dekantiert, was verhältnismässig komplizierte, lange dauernde und/oder häufige Waschvorgänge mit wenigstens einer Pufferlösung erforderlich macht.

Beladene magnetische Mikropartikel werden nach bekannten Verfahren insbesondere abgetrennt, indem sie durch Permanentmagneten an der Wand eines Reaktionsgefässes unter Bildung eines Clusters abgelagert und dort beim Pipettieren oder Dekantieren der Suspensionsflüssigkeit festgehalten werden. Während dem Entfernen der Suspensionsflüssigkeit muss das Magnetfeld aufrechterhalten werden, was in der Regel komplizierte Verfahren und Vorrichtungen zur Folge hat.

In der DE 3926462 A1 wird ein Verfahren und eine Vorrichtung zum Trennen und Waschen von in Flüssigkeitsproben fein verteilt angeordneten magnetischen Feststoffteilchen beschrieben. Die Feststoffteilchen sind mit organischen Substanzen beladen, als Vorstufe für eine photometrische oder radiometrische Auswertung von Patientenproben bei immunoluminometrischen und immunoradiometrischen Tests. Die Flüssigkeitsproben werden in Probenröhrchen einem durch Dauermagnete erzeugten magnetischen Feld ausgesetzt, wobei die magnetischen Feststoffteilchen an der Innenwand der Probenröhrchen angelagert werden. Nach einer vorgegebenen Zeit wird die Restflüssigkeit unter Aufrechterhaltung des Magnetfelds abgesaugt. Der Trenn- und Waschprozess wird vollautomatisch im Durchlaufverfahren durchgeführt, auf einer Förderstrecke für die Probenröhrchen sind mehrere Dauermagnete angeordnet.

Nach der EP 0806665 B1 wird das Trennverfahren zur Abscheidung von magnetischen Mikropartikeln dahingehend verbessert, dass nach einer Variante die unter Einwirkung eines Dauermagneten an der Gefässwand abgeschiedenen Mikropartikel nach dem Absaugen des Spülwassers mit frischem Wasser oder einem frischen Reagenz resuspendiert werden können, was den Reinigungseffekt erheblich steigert. Nach einer bestimmten Verweilzeit werden die Mikropartikel wiederum unter magnetischer Einwirkung der Wand des Reaktionsgefässes angelagert und das Spülwasser erneut abgesaugt. Dieser Vorgang kann mehrmals wiederholt werden.

Die US 6207463 B1 offenbart ein Abtrennen von magnetischen Mikropartikeln aus einer Suspension, indem ein stabförmiger Dauermagnet, welcher bis zur Spitze mit einer Schutzschicht überzogen ist, in die Suspension mit magnetischen Mikropartikeln getaucht wird. Die magnetischen Mikropartikel lagern sich an der Spitze dem Transferelement an und können beim Abheben des Stabes aus der Suspensionslösung entfernt werden, der Cluster haftet am Stab und kann in eine neue flüssige Phase getaucht werden. Dadurch kann das Pipettieren oder Dekantieren weggelassen werden.

Dokument EP 0 479 448 A offenbart eine Vorrichtung gemäß dem Oberbegriff des Anspruchs 1, Proben - und Reagenzbehälter gemäß dem Oberbegriff des Anspruchs 9 und des Verfahren zum automatischen Trennen gemäß dem Oberbegriff des Anspruchs 11.

Der Erfinder hat sich die Aufgabe gestellt, eine Vorrichtung und ein Verfahren zu schaffen, welche einen vollautomatischen Prozessablauf erlauben und leicht, schnell, sicher und kostengünstig anzuwenden sind.

In Bezug auf die Vorrichtung wird die Aufgabe erfindungsgemäss dadurch gelöst, dass eine erste Führung für die Zufuhr von Probenbehältern in x-Richtung und zweite Führungen für die Zufuhr von Reagenzbehältern in y-Richtung zur Prozessarea angeordnet sind, wobei die zweiten Führungen in y-Richtung in einem Winkel α von 30 bis 150° zur x-Richtung verlaufen, ein in x-Richtung hin und her bewegbares Trägerelement einzeln und gesamthaft in z-Richtung hebund senkbare Trägerplatten für matrixförmig angeordnete, magnetische oder magnetisierbare Transferelemente umfasst, die Reagenzbehälter entsprechend dem Raster der Transferelemente durch ein im Winkel α erfolgendes Einführen in die Prozessarea positionierbar und durch Ausstossen in derselben Richtung in einen Abfallsammler verwerfbar sind. Spezielle und weiterbildende Ausführungsformen der Erfindung sind Gegenstand von abhängigen Patentansprüchen.

Transferelemente sind vorzugsweise als Dauermagnetstäbe oder als stabförmige Elektromagneten ausgebildet.

Der unterste, in die Proben- und Reagenzbehälter eintauchende Teil der Transferelemente ist zweckmässig mit einer heb- und absenkbaren, durch eine Relativbewegung bezüglich der Transferelemente aufsetz- und abstreifbaren, vorzugsweise rohr- oder becherförmig ausgebildeten Membrane abgedeckt. Die Membrane kann bei elektromagnetisch arbeitenden Transferelementen weggelassen werden.

Nach dem Stand der Technik laufen getaktete Batches stets eindimensional, in x-Richtung. Erfindungsgemäss verlaufen die getakteten Batches zweidimensional, die Probenbehälter in x-Richtung, die Reagenzbehälter dagegen in y-Richtung. Wie bei Raumkoordinaten üblich, haben die beiden Richtungen bevorzugt einen Winkel α von 90°, sie verlaufen rechtwinklig, ebenfalls zur dritten, vertikalen Raumkoordinate z. Die erste Führung für die Zufuhr der Probenbehälter in x-Richtung ist gegeben, sie verläuft in der gleichen Richtung, wie die Hin- und Herbewegung des Trägerelements. In speziellen Ausführungsformen kann dagegen die y-Richtung für die zweiten Führungen in einem verhältnismässig grossen Winkelbereich variieren. Die zweiten Führungen verlaufen zweckmässig parallel, sie können jedoch auch ausspreizen und/oder ansteigend eine Rutsche bilden. Auch verschlossene Reagenzbehälter haben jedoch spätestens unmittelbar vor der Prozessarea eine horizontale Lage, damit sie mit einem Reagenz beladen werden können oder ein allfälliger Verschluss ohne Auslaufgefahr für das Reagenz aufgerissen oder durchstochen werden kann.

Die Relativbewegung eines Transferelements in deren Längsrichtung gegenüber der Membrane erfolgt vorzugsweise durch unterschiedliches Heben und Senken der betreffenden Trägerplatten bzw. Führungen mit an sich bekannten Mitteln. Selbstverständlich könnte diese Relativbewegung teilweise auch durch Heben und Senken des untenliegenden Trägerblocks für die Reagenzbehälter erfolgen, dies erscheint jedoch als weniger vorteilhaft.

Der Transport der magnetischen Mikropartikel in x-Richtung erfolgt wie erwähnt vorzugsweise an rohr- oder becherförmigen Kavitäten der Membrane im untersten Bereich der Transferelemente, wenn die Hinbewegung der Trägerplatten in x-Richtung erfolgt und die hochgezogene Membrane in den nächstfolgenden Reagenzbehälter abgesenkt werden kann. Diese Membranen sind Verbrauchsmaterial, sie werden an die Einlaufseite der Prozessarea geleitet, positioniert, bei einer Absenkbewegung auf die in x-Richtung hintersten Transferelemente aufgesetzt und mitgenommen. Die Membranen müssen in den Reagenzbehältern und auf etwa halber maximaler Hubhöhe der Transferelemente positionierbar sein, mit und ohne eingeführtes Transferelement. Für die kontinuierliche Zufuhr der Membranen zur Einlaufseite der Prozessarea ist eine dritte Führung vorgesehen, welche in Bezug auf die x-Richtung einen Winkel von vorzugsweise 60 bis 120° hat. Die Membranen sind so gestaltet, dass sie in die Proben- und Reagenzbehälter eingeführt werden können, sie sind also insbesondere in Bezug auf Anzahl und Form der Kavitäten gleich. Weiter sind die Membranen wie die Proben- und Reagenzbehälter vorzugsweise als Spritz- oder Tiefziehteile aus Kunststoff ausgebildet.

Die Behälter und die Membranen sind im wesentlichen streifenförmige, stapelbare Kassetten mit mehreren, dem Raster der Transferelemente in der Trägerplatte entsprechenden becherförmigen Kavitäten.

In Bezug auf das Verfahren zum automatischen Trennen der festen und flüssigen Phase einer Suspension und zum Reinigen der festen Phase wird die Aufgabe erfindungsgemäss dadurch gelöst, dass die Vorwärtsbewegung des Trägerelements in x-Richtung beim Einsatz von Dauermagnetstäben als Transferelemente mit beladenen, hochgezogenen Membranen oder beim Einsatz von stabförmigen Elektromagneten mit eingeschaltetem Strom, die Rückwärtsbewegung entgegen der x-Richtung beim Einsatz von Dauermagnetstäben als Transferelemente ohne Membranen oder beim Einsatz von stabförmigen Elektromagneten mit abgeschaltetem Strom erfolgt. Spezielle und weiterbildende Ausführungsformen des Verfahrens sind Gegenstand von abhängigen Patentansprüchen.

Vorerst werden vorzugsweise die befüllten Probenbehälter intermittierend oder kontinuierlich längsseitig in x-Richtung, die Reagenzbehälter mit unterschiedlichen oder höchstens teilweise gleichen Befüllungen in y-Richtung kontinuierlich stirnseitig an die Prozessarea geführt. Bei jeder Einleitung eines neuen Arbeitszyklus' wird je eine Membrane auf die in x-Richtung hintersten als Dauermagnetstäbe ausgebildeten Transferelemente gestülpt, diese in den an die Prozessarea angelegten Probenbehälter abgesenkt und nach dem Anlagern der magnetischen Mikropartikel an der Membrane die Transferelemente mit der Membrane aus den Suspensionsflüssigkeiten hochgefahren. Das Trägerelement wird um eine Rastereinheit, entsprechend dem Abstand zwischen zwei Reagenzbehältern, in x-Richtung vorwärts verschoben und der partikelfreie Probenbehälter in einen Abfallsammler ausgestossen. Gleichzeitig werden die befüllten Reagenzbehälter in die Prozessarea eingeführt, das Trägerelement mit den Transferelementen in die Reagenzbehälter abgesenkt, die Transferelemente aus den Membranen gezogen, die angelagerten magnetischen Mikropartikel resuspendiert und die Suspension gemischt. Die Transferelemente werden entgegen der x-Richtung um den Abstand a zurückversetzt, während die Membranen in ihrer Position verharren.

Bei jeder Bewegung der Trägerplatten in x-Richtung werden die Membranen um eine Rastereinheit, den Abstand a, mitgenommen und am Ende der Prozessarea in einen Abfallsammler gestossen. Der in x-Richtung letzte, aus der Prozessarea gestossene Reagenzbehälter wird einer beliebigen, an sich bekannten Weiterverwendung zugeführt, beispielsweise einer chemischen Analyse.

Bei als stabförmige Elektromagneten ausgebildeten Transferelementen ohne Membrane wird der Strom zum Beladen mit Mikropartikeln eingeschaltet, zum Resuspendieren ausgeschaltet.

Ein Arbeitszyklus dauert vorzugsweise 2 bis 4 min. Die Dauer eines Arbeitszyklus' ist aus ökonomischen Gründen möglichst tief, gegenwärtig können etwa 2 min. erreicht werden.

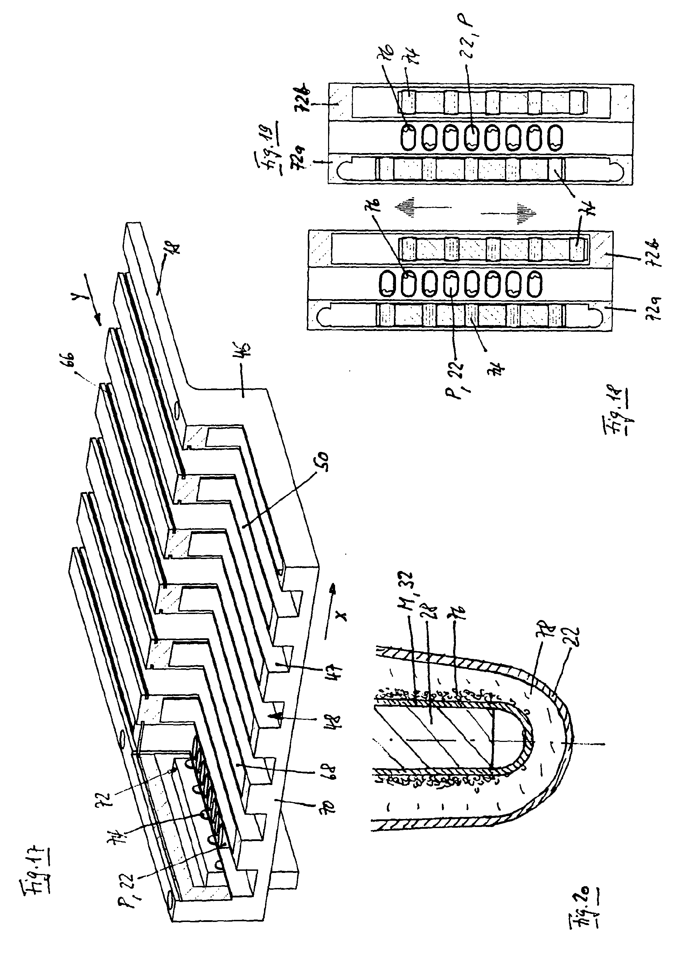

Bei voller Auslastung werden sechs bis zehn Reagenzbehälter gleichzeitig in die Prozessarea gestossen, vorzugsweise haben alle Reagenzbehälter unterschiedliche Reagenzien, wobei auch reines Wasser oder ein organisches Lösungsmittel als Reagenz bezeichnet wird. Selbstverständlich können jedoch auch Sequenzen mit einzelnen sich wiederholenden Reagenzien zusammengestellt werden. Innerhalb desselben Reagenzbehälters haben die Kavitäten stets das gleiche Reagenz. Falls nicht alle Kanäle (48 in Fig. 17) mit Reagenzbehältern besetzt sind, bleiben die in x-Richtung vordersten Kanäle leer. Das Verwerfen der Membranen und die Weiterverwendung des vordersten Reagenzbehälters erfolgt, wie wenn dieser im vordersten Kanal wäre.

Die eingesetzten Mikropartikel haben, wie dies der Name sagt, Dimensionen von einem bis mehreren Mikrometern, sie können auch Bruchteile eines Mikrometers haben und wären dann korrekt mit Nanopartikel zu bezeichnen. Einfachheitshalber wird jedoch für alle Partikelgrössen der Begriff Mikropartikel verwendet. Die Kavitäten der Probe- und Reagenzbehälter haben ein Volumen von in der Regel 1 bis 3 ml.

Die Vorteile der Erfindung können wie folgt zusammengefasst werden.
- Die erfindungsgemässe Vorrichtung kann vollautomatisch betrieben werden.
- Das Verfahren mit den Arbeitszyklen erlaubt, dass alle Kavitäten während des ganzen Prozesses in Aktion sind.
- Zahlreiche Module, in der Regel sechs bis zehn, können gleichzeitig arbeiten, was eine maximale Arbeitsproduktivität bedeutet.
- Die Matrixanordnung erlaubt eine optimal dichte Anordnung, wodurch die Produktivität weiter erhöht wird.
- Das Verfahren erlaubt eine kontinuierliche Prozessierung von Proben.

Die Erfindung wird anhand von in der Zeichnung dargestellten Ausführungsbeispielen, welche auch Gegenstand von abhängigen Patentansprüchen sind, näher erläutert. Es zeigen schematisch:

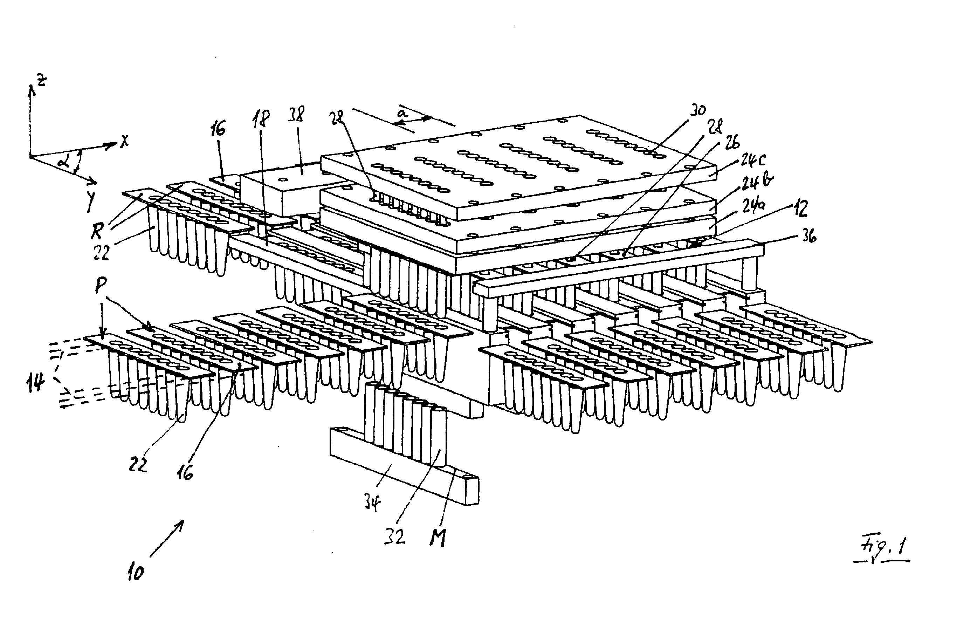

- Fig. 1 eine perspektivische Ansicht der Vorrichtung,
- Fig. 2 einen Vertikalschnitt in x-Richtung durch die Prozessarea,
- Fig. 3 ein Layout der Vorrichtung beim Prozessbeginn,
- Fig. 4 einen Vertikalschnitt in x-Richtung durch die Prozessarea mit angelegtem Probenbehälter,
- Fig. 5 einen anschliessenden Prozessschritt gemäss Fig. 4 mit in dem Probenbehälter eingetauchten Dauermagnet-Stäben mit Membranen,
- Fig. 6 einen nächsten Prozessschritt gemäss Fig. 5 mit in x-Richtung verschobenem Trägerelement,
- Fig. 7 ein Layout der Vorrichtung nach dem Befüllen des ersten Reagenzbehälters,
- Fig. 8 einen weiteren Prozessschritt nach Fig. 6. mit hochgezogenen Dauermagnetstäben und der Membrane im ersten Reagenzbehälter,
- Fig. 9 eine weitere Variante mit in Gegenrichtung zur x-Richtung zurückgefahrenem Trägerelement,
- Fig. 10 einen weiteren Verfahrensschritt gemäss Fig. 9 mit aufgesetzter zweiter Membrane,
- Fig. 11 ein weiterer Verfahrenschritt gemäss Fig. 10 mit abgesenkten Dauermagnetestäben,
- Fig. 12 ein weiteres Layout mit ausgestossenem ersten Reagenzbehälter,
- Fig. 13 ein weiteres Layout der Vorrichtung mit in x-Richtung vorgefahrenem Trägerelement,
- Fig. 14 ein weiterer Verfahrensschritt gemäss Fig. 13 mit in x-Richtung verschobenem Trägerelement,
- Fig. 15 ein weiteres Layout nach mehreren Verfahrensschritten mit dem ersten Reagenzbehälter in Endposition,
- Fig. 16 ein letztes Layout mit ausgestossenem Reagenzbehälter und Membrane,
- Fig. 17 eine teilweise aufgeschnittene perspektivische Ansicht eines Trägerblocks,
- Fig. 18 einen Horizontalschnitt durch einen Magnetmischer,
- Fig. 19 den magnetischen Mischer gemäss Fig. 18 in der andern Position, und
- Fig. 20 den untersten Bereich einer Kavität eines Proben- oder Reagenzbehälters.

Eine perspektivische Ansicht einer erfindungsgemässen Vorrichtung 10 mit den vorzugsweise rechtwinkligen Raumkoordinaten x, y und z umfasst im wesentlichen eine zentrale Prozessarea 12, wo die Trennungs- und Reinigungsprozesse stattfinden, lediglich gestrichelt angedeutete erste Führungen 14 für Probenbehälter P und zweite Führungen 18 für Reagenzbehälter R. Die ersten Führungen 14 in x-Richtung und die zweiten Führungen 18 in y-Richtung haben einen Winkel α von 90°, verlaufen also rechtwinklig. Sowohl die Proben P als auch die Reagenzbehälter R sind im wesentlichen streifenförmig ausgebildet und haben rohr- oder becherförmige Kavitäten 22, welche im Spritzgussverfahren aus Kunststoff hergestellt werden, wobei die Probenbehälter P und Reagenzbehälter R identisch sind. Ein umlaufender Flansch 16 stabilisiert nicht nur die Kavitäten 22, er dient auch der Führung und Halterung.

Die Prozessarea 12 wird in ihrer horizontalen Ausdehnung weitgehend durch ein Tragelement 24 begrenzt, welches aus drei Tragplatten 24a, 24b und 24c besteht. Mit der untersten Tragplatte 24a wird das ganze Tragelement 24 in z-Richtung gehoben und gesenkt, der Antrieb und die Steuerung dazu sind - wie der Antrieb für die Zufuhr der Probebehälter P und der Reagenzbehälter R mit an sich bekannten Mitteln durchgeführt. Die Tragplatten 24b und 24c können gemeinsam, jedoch auch einzeln angehoben und gesenkt werden, wobei eine relative Verschiebung resultiert. In diesem Fall werden die als Dauermagnetstäbe ausgebildeten Transferelemente 28 in die Membranen M gestossen oder daraus herausgezogen.

Das Tragelement 24 hat einen vorgegebenen Raster von Bohrungen 30, welche von Dauermagnetstäben 28 durchgriffen werden. Diese haben umlaufende Krägen, welche auf der Trägerplatte 24c aufliegen. Die Membranen M sind grundsätzlich wie die Probenbehälter P und Reagenzbehälter R ausgebildet, die Kavitäten 32 sind jedoch in der Regel zylinderrohrförmig ausgebildet. Mit einer dritten Führung, welche der Übersichtlichkeit wegen nicht dargestellt ist, wird von vorne links eine vorbereitete Membrane M zugeführt. Die Membranen M werden am Eingang zur Prozessarea 12 aufgenommen und bei jedem Arbeitszyklus um die Rasterdistanz a in x-Richtung befördert. Dies erfolgt mittels Verschiebung auf einem horizontalen Schienenpaar 36.

Bei jedem Arbeitszyklus erreicht ein abgefüllter Probenbehälter P längsseitig die Prozessarea 12. Am in x-Richtung vordersten Probenbehälter P werden bei eingeführten Dauermagnetstäben 28 an der Membrane M bzw. deren Kavitäten 32 magnetische Mikropartikel (76 in Fig. 20) angelagert. Diese werden über die Membranen M stufenweise von Reagenzbehälter R zu Reagenzbehälter R gefördert, wobei die angelagerten Mikropartikel in jedem Arbeitszyklus resuspendiert werden können. Die Probenbehälter P werden in y-Richtung ausgestossen und in einem nicht dargestellten Abfallbehälter gesammelt.

Die in y-Richtung zugeführten Reagenzbehälter R sind ebenfalls in-line befüllt worden. Falls vorkonfektionierte, befüllte Reagenzbehälter R eingesetzt werden, werden diese unmittelbar vor der Prozessarea 12 im Deckelbereich aufgerissen oder durchstochen, damit eine Membrane M bzw. die Dauermagnetstäbe 28 zugeführt werden können. Die Einrichtungen zum Befüllen oder Öffnen sind in oder an einem ebenfalls in z-Richtung heb- und senkbaren Gehäuse 38 angeordnet. Im Arbeitstakt werden vorliegend sechs Reagenzbehälter R in die Prozessarea 12 eingeschoben und die verbrauchten Behälter in y-Richtung in einen Abfallsammler ausgestossen. Es werden weder Probenbehälter P noch Reagenzbehälter R in x-Richtung durch die Prozessarea 12 geführt.

In jedem Arbeitszyklus von etwa drei Minuten Dauer werden 48 Proben gleichzeitig getrennt oder gereinigt bzw. gewaschen. Pro Arbeitszyklus verlassen jeweils acht Proben die Prozessarea 12, während einer Stunde also etwa 160 Proben.

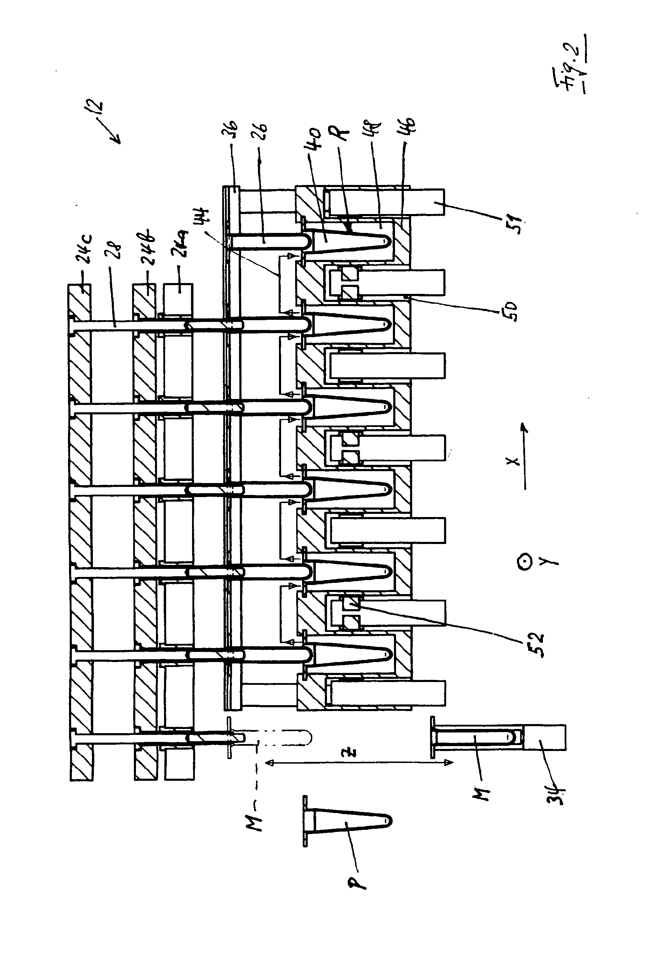

In Fig. 2 ist die Prozessarea 12 zu Beginn eines Arbeitszyklus dargestellt. Die Tragplatten 24a und 24b sind soweit angehoben, dass die Membranen M oberhalb dem Niveau der Suspension 40 in den Reagenzbehältern R liegen. Die oberste Tragplatte 24c ist soweit gehoben, dass die Dauermagnetstäbe 28 praktisch aus den Membranen M gezogen sind. Der in x-Richtung, der Vorschubrichtung, vorderste Probenbehälter P liegt noch ausserhalb der Prozessarea 12.

Eine Halterung 34 mit einer Membrane M ist an die Prozessarea 12 gefahren, die Membrane M kann in z-Richtung über die Dauermagnetstäbe 28 gestülpt werden. Dies ist der erste Prozessschritt von einem Arbeitszyklus. Der Weg der Membranen M durch die ganze Prozessarea 12 in x-Richtung ist mit Pfeilen 44 angedeutet.

Im Trägerblock 46 verlaufen senkrecht zur x-Richtung beidseits offene Kanäle 48 für die von hinten eingeschobenen, in Arbeitsposition entsprechend dem Raster der Dauermagnetstäbe 28 positionierte und nach vorne ausgestossenen Reagenzbehälter R. Alternierend zu seitlich und nach oben offenen Kanälen 48 sind Aussparungen 50 ausgebildet, in welchen in y-Richtung, d.h. senkrecht zur Blattebene, hin- und herschiebbare Balken 52 mit später im Detail gezeigten Dauermagneten zum Mischen der Suspension 40 angeordnet sind.

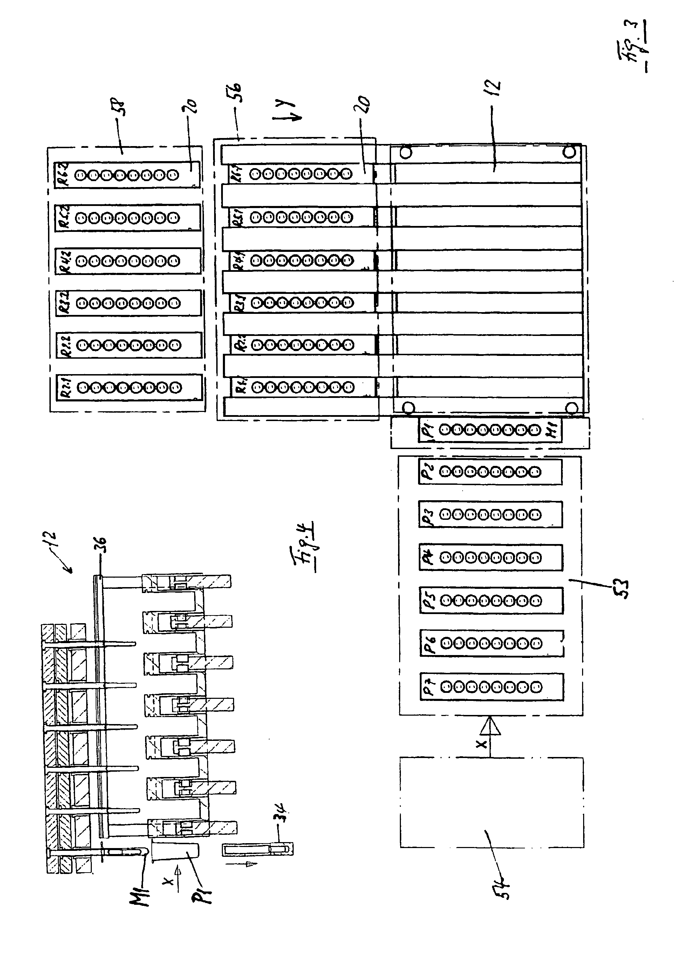

Ein Layout gemäss Fig. 3 zeigt - von oben gesehen - die Prozessarea 12, in welcher weder Probebehälter P, noch Reagenzbehälter R noch Membranen M eingeführt sind. Ein entsprechend dem Raster in x-Richtung im Abstand a anliegende Probenbehälter P1 wird von einer von unten auf die Dauermagnetstäbe 28 gestülpte Membrane M1 verdeckt. Eine Pufferstrecke 53 enthält die Probenbehälter P2 bis P7, welche von der Befüllstation 54 eingespeist werden.

In y-Richtung sind sechs Reaganzbehälter R1.1 bis R6.1 stirnseitig an die Prozessarea 12 gestossen. In dieser Position werden die Reagenzbehälter R geöffnet oder mit Reagenzien befüllt. Die Reagenzbehälter R einer Pufferstrecke 58 sind mit R2.1 bis R6.2 bezeichnet.

Fig. 4 zeigt die Situation gemäss Fig. 3 im Vertikalschnitt in x-Richtung. Die in x-Richtung hintersten Dauermagnetstäbe 28 haben von unten die Membrane M1 übergestülpt. In x-Richtung ist der vorderste Probenbehälter P1 an die Prozessarea 12 angestellt. Dieser liegt exakt unter der Membrane M1. Die Halterung 34 der Membrane M1 wird nach dem Heben der Membrane M1 ausgestossen und entsorgt, was mit einem Pfeil angedeutet ist.

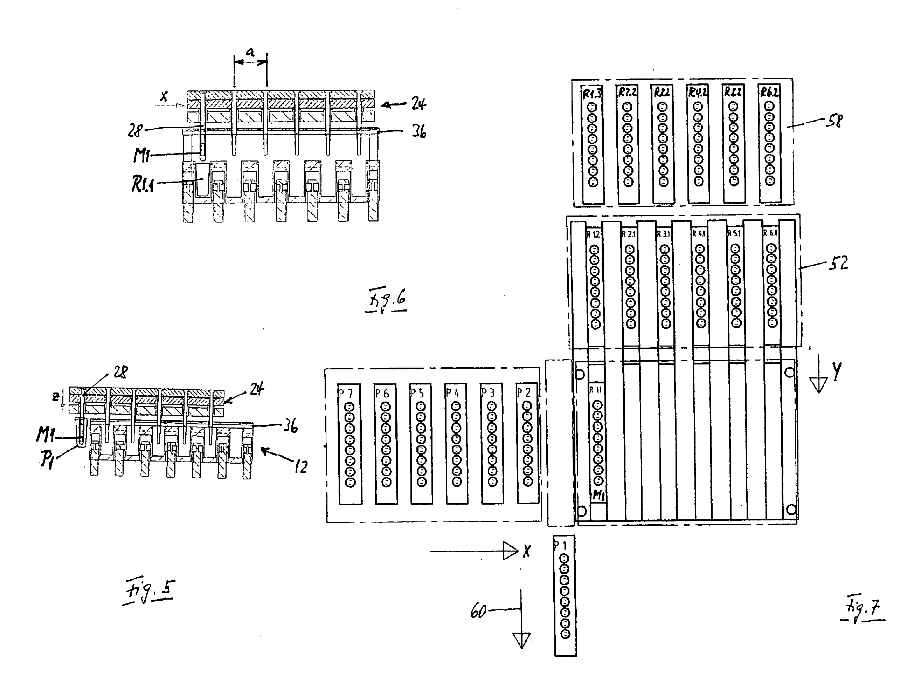

In Fig. 5 ist das Schienenpaar 36 und das Tragelement 24 in Richtung z vollständig abgesenkt, die Membranen M1 tauchen mit eingeführtem Dauermagnetstab 28 in die Suspension des längsseitig an die Prozessarea 12 angelegten Probenbehälters P1 ein. Unter der Einwirkung der Dauermagnetstäbe 28 setzen sich die magnetischen Mikropartikel der Suspension an der Membrane M1 fest und bleiben beim Hochfahren des Schienenpaars 36 und des Tragelements 24 an der Membrane 26 hängen.

Fig. 6 zeigt die in gleichem Masse hochgefahrenen Schienenpaar 36 und Tragelement 24. In dieser Position ist das Tragelement 24 um den Rasterabstand a in x-Richtung verschoben worden. Die in x-Richtung hintersten Dauermagnetstäbe 28 mit der übergestülpten Membrane M1 sind nun exakt oberhalb des inzwischen eingeschobenen Reagenzbehälters R.1.1. Die Membrane M1 ist gemäss Fig. 6 im Schienenpaar 36 in diese Position geschoben worden. Der Reagenzbehälter 1.2 ist gemäss Fig. 7 von der Pufferstrecke 58 in die Pufferstrecke 52 nachgerutscht, wo sie mit Reagenzien befüllt oder die Versiegelung eingestossen wird. In die Pufferstrecke 58 ist Reagenzbehälter R1.3 nachgestossen worden.

Gegenüber dem Layout gemäss Fig. 3 ist der Probenbehälter P1 in y-Richtung weggestossen worden und fällt in einen nicht dargestellten Abfallsammler. Dies ist mit einem Pfeil 60 angedeutet.

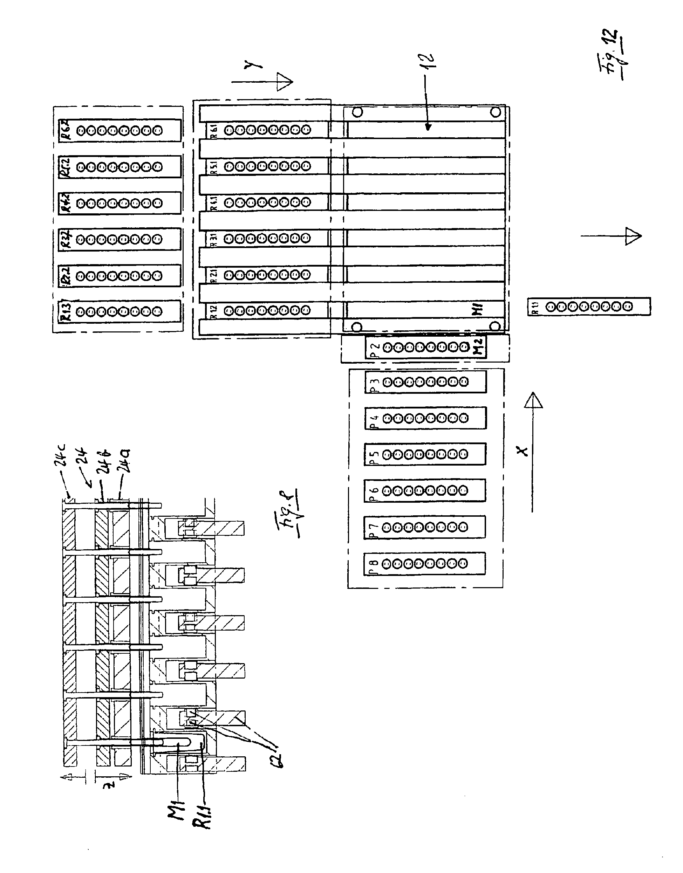

In Fig. 8 wird gezeigt, dass das Trägerelement 24 mit den Trägerplatten 24a, 24b und 24c so weit abgesenkt wird, dass die erste Membrane M1 in den Reagenzbehälter R1.1 eingetaucht ist. Unter magnetischer Einwirkung sammeln sich die Mikropartikel auf der Oberfläche der Membrane M1. Nach einer Reaktionszeit von etwa 1 Minute wird die Trägerplatte 24c mit den Dauermagnetstäben 28 aus der Membrane M1 gezogen. Nun wird eine magnetische Mischeinrichtung 62 in Gang gesetzt. Die magnetischen Mikropartikel lösen sich durch die Mischeinrichtung 62 von der Membrane M1 und werden resuspendiert.

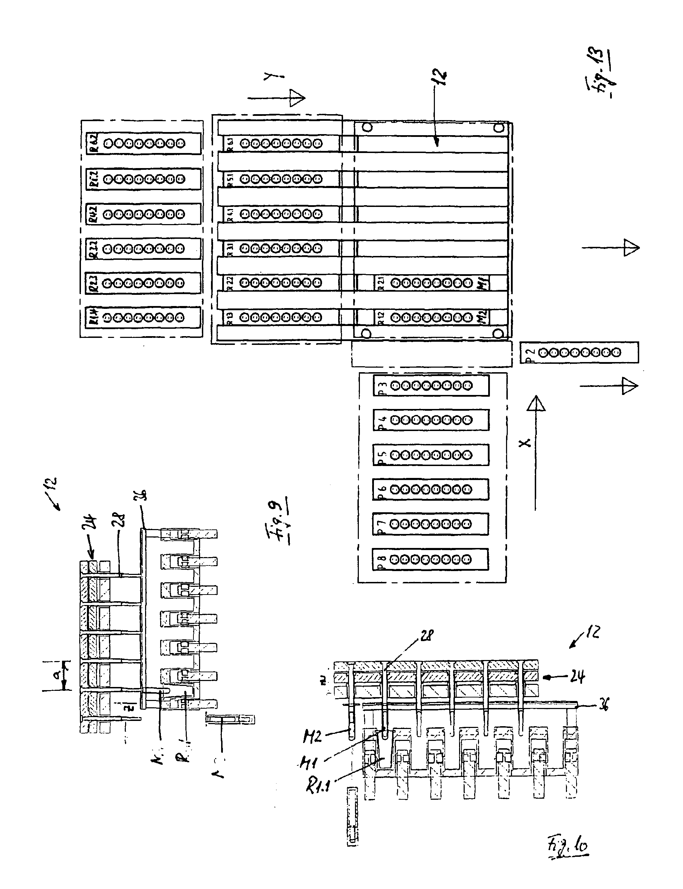

Im nächsten Arbeitsschritt gemäss Fig. 9 wird das komplette Trägerelement 24 hochgefahren, bis die Dauermagnetstäbe 28 ganz aus der mit dem Schienenpaar 36 ebenfalls hochgefahrenen Membrane M1 entfernt sind. Dann wird eine zweite Membrane M2 bereitgestellt. Nun kann das Trägerelement 24 entgegen der x-Richtung um eine Rastereinheit a zurückgefahren werden, die in x-Richtung hintersten Dauermagnetstäbe 28 liegen nun exakt oberhalb der bereitgestellten zweiten Membrane M2.

Im anschliessenden Verfahrensschritt nach Fig. 10 wird das Tragelement 24, bestehend aus den drei Tragplatten 24a, 24b und 24c, in z-Richtung heruntergefahren, bis die in z-Richtung zweithintersten Dauermagnetstäbe 28 in die ersten Membranen M1 in Arbeitsposition eingetaucht sind. Gleichzeitig werden die zweiten Membranen M2 auf die Dauermagnetstäbe 28 gestülpt.

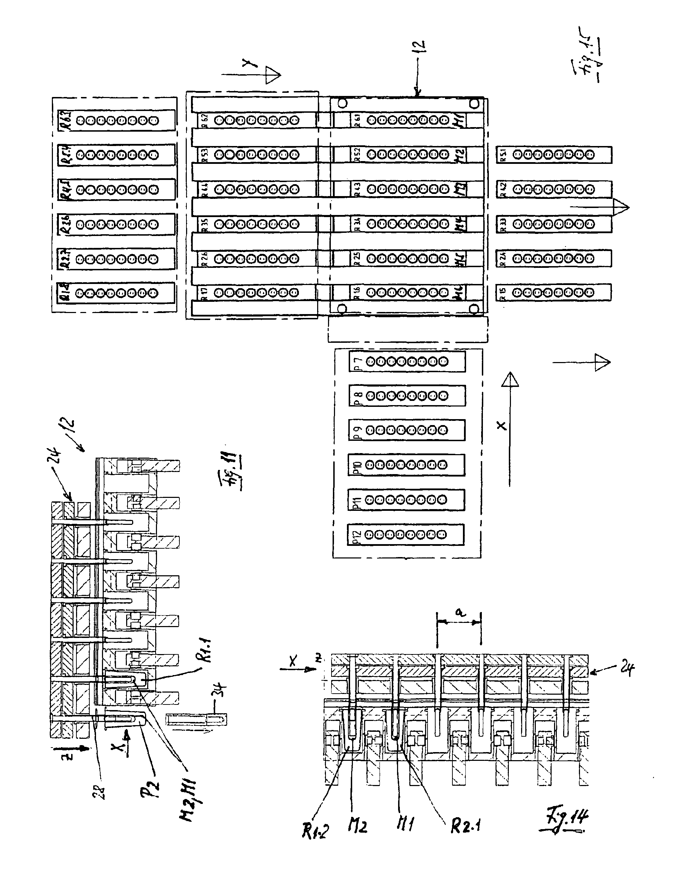

In Fig. 11 sind drei Prozessschritte vereinigt. Vorerst wird die Halterung 34 für die zweite Membrane M2 entfernt, was mit einem Pfeil charakterisiert wird. Dann wird der in x-Richtung zweitvorderste Probenbehälter P2 an die Prozessarea 12 geführt und schliesslich das Tragelement 24 soweit abgesenkt, dass die erste Membrane M1 in den ersten Probenbehälter P1.1 und die zweite Membrane M2 in den zweitvordersten Probenbehälter P2 taucht und die entsprechenden Dauermagnetstäbe 28 in die Membrane M2 abgesenkt sind. Nun lagern sich die Mikropartikel an den beiden ersten Membranen M1, M2 an. Sie können beim Hochziehen der Dauermagnetstäbe 28 aus der flüssigen Phase der Suspension entfernt werden. Nun kann der mikropartikelfreie Reagenzbehälter R1.1 in y-Richtung aus der Prozessarea 12 gestossen werden, was im Layout von Fig. 12 dargestellt ist. In der Prozessarea 12 verbleibt lediglich die erste Membrane M1.

Nach dem folgenden, in Fig. 13 dargestellten Layout wird auch der zweite Probenbehälter P2 in y-Richtung ausgestossen und dem Abfallsammler zugeführt. In die Prozessarea 12 werden die Prozessbehälter R1.2 und R2.1 nachgestossen und unterhalb der Membranen M1 und M2 positioniert.

Gemäss Fig. 14 wird nun das Tragelement 24 mit den Dauermagnetstäben 28 und den Membranen M in x-Richtung um eine Rastereinheit a vorwärts geschoben. Und dann als Ganzes in z-Richtung abgesenkt, bis die Membranen M1 und M2 in die beiden ersten Reagenzbehälter R1.2 und R2.1 positioniert sind. Die magnetischen Mikropartikel werden nun an der Aussenwand der Membranen M1 und M2 gesammelt und lagern sich an. In diesem mit Fig. 9 begonnenen neuen Arbeitszyklus wird nun weitergefahren wie im vorhergehenden Arbeitszyklus: Hochheben der Dauermagnetstäbe 28, Mischen der sich lösenden magnetischen Mikropartikel usw. Jeder neue Arbeitszyklus wird mit dem Aufsetzen einer Membrane M und der Entnahme der Proben aus dem in x-Richtung vordersten, längsseitig an die Prozessarea 12 angestellten Probenbehälter P begonnen.

Im Layout gemäss Fig. 15 hat die Membrane M1 die in x-Richtung letzte Arbeitsposition in der Prozessarea 12 erreicht. Die Behandlung der Proben ist abgeschlossen, alle vorgesehenen Operationen sind anschliessend durchgeführt. Erst in dieser Konfiguration ist die Vorrichtung voll betriebsfähig.

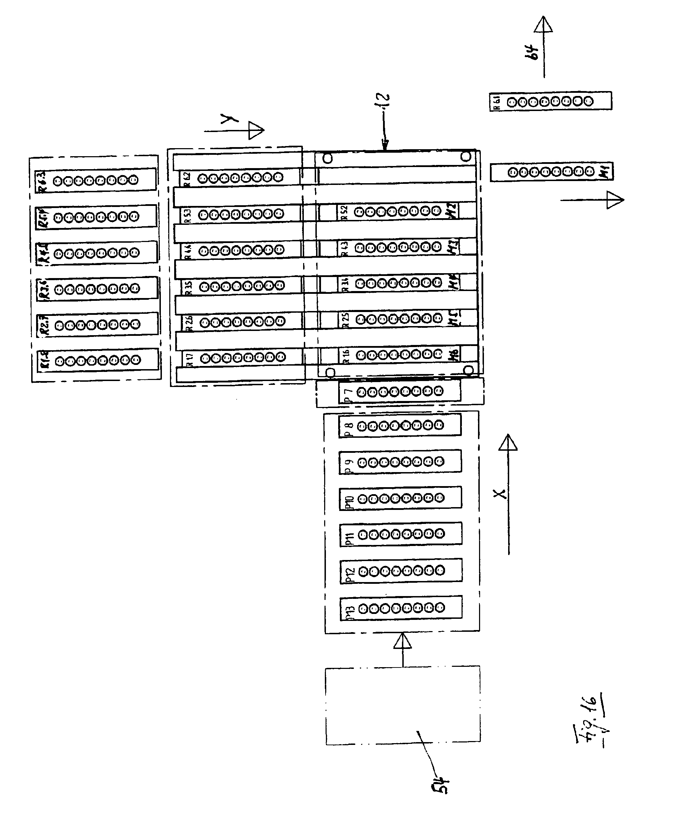

Im Layout gemäss Fig. 16 wird gezeigt, dass die erste Membrane M1, welche alle Arbeitszyklen durchlaufen hat, nach dem Ausstossen aus der Prozessarea 12 dem Abfallsammler zugeführt wird. Der Reagenzbehälter 6.1 mit dem Resultat der sechs Arbeitszyklen dagegen wird der Verwendung zugeführt, was mit einem Pfeil 64 angedeutet ist.

Fig. 17 zeigt einen von der Übersichtlichkeit wegen aufgebrochenen Trägerblock 46 der Prozessarea 12. Es sind sechs durchgehende, oben offene Kanäle 48 für den Durchlauf der Reagenzbehälter R ausgebildet. Beim Durchlauf gleiten die Reagenzbehälter R mit ihrem umlaufenden Flansch 16 in gegenüberliegenden Nuten 66 in den Seitenwänden 47 der Kanäle 48.

Zwischen den Kanälen 48 des Trägerblocks 46 sind weitere Aussparungen 68 vorgesehen, welche mit den Kanälen 48 alternieren und auf der Stirnseite 70 des Trägerblocks 46 geschlossen sind. Die Aussparungen 68 dienen der Aufnahme von in y-Richtung hin- und her schiebbaren Balken 72 mit integrierten Dauermagneten 74. Die Dauermagnete 74 sind im Bereich der Kavitäten 22 der Reagenzbehälter R angeordnet und dienen dem Mischen der resuspendierten magnetischen Mikropartikel 76.

Wie aus Fig. 18 und 19 ersichtlich, sind die hin- und herbewegbaren Balken 72a, 72b beidseits der Kavitäten 22 eines Reagenzbehälters R angeordnet. Die Dauermagnete 74 sind in doppeltem Abstand der Kavitäten 22 angeordnet. Beim Einschalten entsteht eine Relativbewegung zwischen den Dauermagneten 74 und den Kavitäten 22. Die einseitig angelagerten Mikropartikel 76 wechseln die Seite, wodurch ein wirkungsvoller Mischeffekt entsteht. Die Wirkung kann verbessert werden, indem die Kavitäten 22 elliptisch, oval oder rechteckig mit runden Kurzseiten ausgebildet sind.

In Fig. 20 ist der untere Bereich einer Kavität 22 mit einer Suspension 78 stark vergrössert dargestellt. Der unterste Bereich eines Dauermagnetstabs 28 ist mit einer becherförmigen Membrane M umhüllt. Die Dauermagneten 28 bewirken, dass sich die Mikropartikel 76 an der Membrane M bzw. deren Kavitäten 32 anlagern. Wird die Membrane M zusammen mit den Dauermagnetstäben 28 entfernt, werden die Mikropartikel 76 aus der praktisch partikelfreien Suspension 78 abgehoben. Wird dagegen der Dauermagnetstab 28 entfernt und die Membrane M belassen, lösen sich die Mikropartikel 76 wieder von der Membrane M. Durch Mischen, beispielsweise wie in Fig. 18 und 19 gezeigt, kann der Ablöseprozess beschleunigt und der Mischeffekt verbessert werden.

## Patentansprüche

1. Vorrichtung (10) zum automatischen Trennen der festen und flüssigen Phase einer Suspension (78) und zum Reinigen von mit organischen, insbesondere molekularbiologischen oder biochemischen Substanzen beladenen, magnetischen Mikropartikeln (76), welche Vorrichtung (10) eine Prozessarea (12) mit getaktet wandernden Einrichtungen für den Transport der magnetischen Mikropartikel (76) in x-Richtung umfasst,
**dadurch gekennzeichnet, dass**
eine erste Führung (14) für die Zufuhr von Probenbehältern (P) in x-Richtung und zweite Führungen (18) für die Zufuhr von Reagenzbehältern (R) in y-Richtung zur Prozessarea (12) angeordnet sind, wobei die zweiten Führungen (18) in y-Richtung in einem Winkel (α) von 30 bis 150° zur x-Richtung verlaufen, ein in x-Richtung hin und her bewegbares Trägerelement (24) einzeln und gesamthaft in z-Richtung heb- und senkbare Trägerplatten (24a,24b,24c) für matrixförmig angeordnete, magnetische oder magnetisierbare Transferelemente (28) umfasst, die Reagenzbehälter (R) entsprechend dem Raster der Transferelemente (28) durch ein im Winkel (α) erfolgendes Einführen in die Prozessarea (12) positionierbar und durch Ausstossen in derselben Richtung in einen Abfallsammler verwerfbar sind.

2. Vorrichtung (10) nach Anspruch 1, **dadurch gekennzeichnet, dass** die Transferelemente (28) als vorzugsweise stabförmige Dauermagnete oder Elektromagnete ausgebildet sind.

3. Vorrichtung nach Anspruch 1 oder 2, **dadurch gekennzeichnet, dass** der unterste, in die Proben- (P) und Reagenzbehälter (R) eintauchende Teil der Transferelemente (28) mit einer heb- und absenkbaren, durch eine Relativbewegung bezüglich der Transferelemente (28) aufsetz- und abstreifbaren, vorzugsweise rohr- oder becherförmig ausgebildeten Membrane (M) abgedeckt ist.

4. Vorrichtung (10) nach einem der Ansprüche 1 bis 3, **dadurch gekennzeichnet, dass** der Winkel (α) zwischen der x- und y-Richtung 90° beträgt.

5. Vorrichtung (10) nach einem der Ansprüche 1 bis 4, **dadurch gekennzeichnet, dass** die Relativbewegung eines Transferelements (28) zur entsprechenden Membrane (M) in deren Längsrichtung (z) durch unterschiedliches Heben oder Senken der entsprechenden Trägerplatten (24b,24c) und der Membranen (M) erfolgt.

6. Vorrichtung (10) nach einem der Ansprüche 1 bis 5, **dadurch gekennzeichnet, dass** eine dritte Führung zur kontinuierlichen Zufuhr der Membranen (M), in einem Winkel (β) von 60 bis 120° bezüglich der x-Richtung angeordnet ist.

7. Vorrichtung (10) nach einem der Ansprüche 1 bis 6, **dadurch gekennzeichnet, dass** in der Prozessarea (12) ein Trägerblock (46) mit senkrecht zur x-Richtung verlaufenden Kanälen (48) für die Reagenzbehälter (R) angeordnet ist, welche je zwei auf gleichem Niveau gegenüberliegende, stirnseitig offene Horizontalnuten (66) in den Seitenwänden (47) aufweisen.

8. Vorrichtung (10) nach Anspruch 6, **dadurch gekennzeichnet, dass** in parallel zu den Kanälen (48) verlaufenden Aussparungen (50) horizontal hin- und her verschiebbare Balken (72) mit im Bereich der absenkbaren Transferelemente (28) angeordneten Dauermagnete (74) zum Resuspendieren und Mischen der Mikropartikel (76) angeordnet sind.

9. Proben- und Reagenzbehälter (P,R) sowie Membranen (M) zum Einsatz in einer Vorrichtung (10) nach einem der Ansprüche 1 bis 8, **dadurch gekennzeichnet, dass** sie als im wesentlichen streifenförmige, stapelbare Kassetten mit mehreren dem Raster der Transferelemente (28) im Trägerelement (24) entsprechende becherförmige Kavitäten (22,36) ausgebildet sind.

10. Proben- und Reagenzbehälter (P,R) nach Anspruch 9, **dadurch gekennzeichnet, dass** die vorzugsweise sechs bis zehn Kavitäten (22) im Querschnitt flach oder oval ausgebildet sind, wobei ihre querschnittlichen Innenabmessungen vorzugsweise nur wenig über den entsprechenden Abmessungen der Transferelemente (28) oder der aufgezogenen Membranen (M) liegen.

11. Verfahren zum automatischen Trennen der festen und flüssigen Phase einer Suspension und zum Reinigen der festen Phase mit einer Vorrichtung (10), Probenbehältern (P) und Reagenzbehältern (R) nach einem der Ansprüche 1 bis 10,
**dadurch gekennzeichnet, dass**
die Vorwärtsbewegung des Trägerelements (24) in x-Richtung beim Einsatz von Dauermagnetstäben als Transferelemente (28) mit beladenen, hochgezogenen Membranen (M) oder beim Einsatz von stabförmigen Elektromagneten mit eingeschaltetem Strom, die Rückwärtsbewegung entgegen der x-Richtung beim Einsatz von Dauermagnetstäben als Transferelemente (28) ohne Membranen (M) oder beim Einsatz von stabförmigen Elektromagneten mit abgeschaltetem Strom erfolgt.

12. Verfahren nach Anspruch 11, **dadurch gekennzeichnet, dass** vorerst die befüllten Probenbehälter (P) intermittierend oder kontinuierlich längsseitig in x-Richtung und die Reagenzbehälter (R) mit unterschiedlichen oder höchstens teilweise gleichen Befüllungen in y-Richtung kontinuierlich stirnseitig an die Prozessarea (12) geführt, bei jeder Einleitung eines neuen Arbeitszyklus' je eine Membrane (M) auf die in x-Richtung hintersten Transferelemente (28) gestülpt, diese in den an die Prozessarea (12) angelegten Probenbehälter (P) abgesenkt und nach dem Anlagern der magnetischen Mikropartikel (76) an der Membrane (M) die Transferelemente (28) mit der Membrane (M) aus den Suspensionsflüssigkeiten hochgefahren, das Trägerelement (24) um eine Rastereinheit, entsprechend dem Abstand (a) zwischen zwei Reagenzbehältern (R), in x-Richtung vorwärts verschoben, der partikelfreie Probenbehälter (P) in einen Abfallsammler ausgestossen, die befüllten Reagenzbehälter (R) gleichzeitig in die Prozessarea (12) eingeführt, das Trägerelement (24) mit den Transferelementen (28) in die Reagenzbehälter (R) abgesenkt, die Transferelemente (28) aus den Membranen (M) gezogen, die angelagerten magnetischen Mikropartikel (76) resuspendiert, die Suspension (78) gemischt, die Transferelemente (28) entgegen der x-Richtung um den Abstand (a) zurückversetzt, während die Membranen (M) in ihrer Position verharren.

13. Verfahren nach Anspruch 11 oder 12, **dadurch gekennzeichnet, dass** bei jeder Bewegung des Trägerelements (24) in x-Richtung die Membranen (M) um eine Rastereinheit mitgenommen und am Ende der Prozessarea (12) in einen Abfallsammler gestossen werden.

14. Verfahren nach einem der Ansprüche 11 bis 13, **dadurch gekennzeichnet, dass** der in x-Richtung letzte, aus der Prozessarea (12) gestossene Reagenzbehälter (R) einer Weiterverwendung zugeführt wird.

15. Verfahren nach einem der Ansprüche 11 bis 14, **dadurch gekennzeichnet, dass** ein Arbeitszyklus 2 bis 4 min. dauert.

16. Verfahren nach einem der Ansprüche 11 bis 15, **dadurch gekennzeichnet, dass** Reagenzbehälter (R) mit unterschiedlichen Reagenzien, jedoch mit in allen Kavitäten (22) desselben Reagenzbehälters (R) gleichen Reagenzien eingesetzt werden. '

## Claims

1. Device (10) for automatic separation of the solid and liquid phases of a suspension (78) and for cleaning magnetic microparticles (76) loaded with organic substances, in particular molecular biological or biochemical substances, which device (10) comprises a process area (12) with clocked migrating apparatuses for the transport of the magnetic microparticles (76) in the x-direction,
**characterized in that**
a first guide (14) is arranged for the supply of sample containers (P) in the x-direction and second guides (18) are arranged for the supply of reagent containers (R) in the y-direction in respect of the process area (12), wherein the second guides (18) run in the y-direction at an angle (α) of 30 to 150° in respect of the x-direction; a support element (24), which can be moved to and fro in the x-direction, comprises support plates (24a, 24b, 24c) for magnetic or magnetizable transfer elements (28) that are arranged in the form of a matrix, which support plates can be raised and lowered in the z-direction, individually and together, the reagent containers (R) can be positioned in accordance with the grid of the transfer elements (28) by being inserted into the process area (12) at the angle (α) and can be disposed of into a waste collector by ejection in the same direction.

2. Apparatus (10) according to Claim 1, **characterized in that** the transfer elements (28) are designed as preferably rod-shaped permanent magnets or electromagnets.

3. Apparatus according to Claim 1 or 2, **characterized in that** the lowermost part of the transfer elements (28), which dips into the sample (P) and reagent (R) containers, is covered by a membrane (M), which is preferably of a tube-shaped or cup-shaped design, that can be raised and lowered, and relative motion in respect of the transfer elements (28) can attach and strip off said membrane.

4. Apparatus (10) according to one of Claims 1 to 3, **characterized in that** the angle (α) between the x-direction and y-direction is 90°.

5. Apparatus (10) according to one of Claims 1 to 4, **characterized in that** the relative motion of a transfer element (28) towards the corresponding membrane (M), in the longitudinal direction (z) thereof, is brought about by unequal raising or lowering of the corresponding support plates (24b, 24c) and of the membranes (M).

6. Apparatus (10) according to one of Claims 1 to 5, **characterized in that** a third guide for continuous supply of the membranes (M) is arranged at an angle (β) of 60 to 120° in respect of the x-direction.

7. Apparatus (10) according to one of Claims 1 to 6, **characterized in that**, arranged in the process area (12), there is a support block (46) with channels (48) for the reagent containers (R), which channels run perpendicularly to the x-direction and each have two horizontal grooves (66) in the side walls (47), said grooves being open on the end side and situated opposite to one another at the same level.

8. Apparatus (10) according to Claim 6, **characterized in that** beams (72) that can be displaced to and fro horizontally are arranged in recesses (50), which run parallel to the channels (48), and said beams have permanent magnets (74) for resuspending and mixing the microparticles (76), which permanent magnets are arranged in the region of the transfer elements (28) that can be lowered.

9. Sample and reagent containers (P, R) and membranes (M) for use in an apparatus (10) according to one of Claims 1 to 8, **characterized in that** they are designed as substantially strip-shaped, stackable cassettes with a plurality of cup-shaped cavities (22, 36) corresponding to the grid of the transfer elements (28) in the support element (24).

10. Sample and reagent containers (P, R) according to Claim 9, **characterized in that** the preferably six to ten cavities (22) have a flat or oval cross section, wherein their cross-sectional internal dimensions are preferably only slightly larger than the corresponding dimensions of the transfer elements (28) or the pulled-on membranes (M).

11. Method for automatic separation of the solid and liquid phases of a suspension and for cleaning the solid phase using an apparatus (10), sample containers (P) and reagent containers (R) according to one of Claims 1 to 10,
**characterized in that**
there is forward motion of the support element (24) in the x-direction when using permanent magnet bars as transfer elements (28) with loaded, pulled-up membranes (M) or when using bar-shaped electromagnets with the current switched on and there is backward motion counter to the x-direction when using permanent magnet bars as transfer elements (28) without membranes (M) or when using bar-shaped electromagnets with the current switched off.

12. Method according to Claim 11, **characterized in that**, first of all, the filled sample containers (P) are guided, intermittently or continuously, along the longitudinal side in the x-direction to the process area (12) and the reagent containers (R) with different fillings, or at most partly the same fillings, are guided, continuously, along the end side in the y-direction to the process area (12); one membrane (M) is each case put over the backmost transfer elements (28) in the x-direction each time a new work cycle is started, said elements are lowered into the sample containers (P) attached to the process area (12) and the transfer elements (28) with the membrane (M) are raised out of the suspension liquids after the adsorption of the magnetic microparticles (76) on the membrane (M); the support element (24) is advanced forward in the x-direction by one grid unit corresponding to the distance (a) between two reagent containers (R); the particle-free sample container (P) is ejected into a waste collector; the filled reagent containers (R) are simultaneously introduced into the process area (12); the support element (24) with the transfer elements (28) is lowered into the reagent containers (R); the transfer elements (28) are pulled out of the membranes (M); the adsorbed magnetic microparticles (76) are resuspended; the suspension (78) is mixed; and the transfer elements (28) are returned by the distance (a) counter to the x-direction while the membranes (M) remain in their positions.

13. Method according to Claim 11 or 12, **characterized in that** the membranes (M) are carried along by one grid unit during every motion of the support element (24) in the x-direction and are ejected into a waste collector at the end of the process area (12).

14. Method according to one of Claims 11 to 13,
**characterized in that** the last reagent container (R) in the x-direction, which was ejected from the process area (12), is forwarded for further use.

15. Method according to one of Claims 11 to 14,
**characterized in that** a work cycle lasts 2 to 4 min.

16. Method according to one of Claims 11 to 15,
**characterized in that** reagent containers (R) with different reagents are used, but all cavities (22) of the same reagent container (R) have the same reagents.

## Revendications

1. Dispositif (10) de séparation automatique de la phase solide et de la phase liquide d'une suspension (78) et de nettoyage de microparticules magnétiques (76) chargées de substances organiques et en particulier de substances moléculaires biologiques ou biochimiques, le dispositif (10) comprenant une zone de traitement (12) dotée de dispositifs de transport par déplacement cadencé des microparticules magnétiques (76) dans la direction x,
**caractérisé en ce que**
un premier guide (14) est disposé pour amener vers la zone de traitement (12) des récipients d'échantillon (P) dans la direction x et des deuxièmes guides (18) sont disposés pour amener vers la zone de traitement des récipients de réactif (R) dans la direction y, les deuxièmes guides (18) qui s'étendent dans la direction y formant un angle (α) de 30 à 150° par rapport à la direction x,
**en ce qu'**un élément de support (24) qui peut être déplacé en va-et-vient dans la direction x comporte pour des éléments (28) de transfert magnétiques ou magnétisables disposés en matrice des plaques de support (24a, 24b, 24c) qui peuvent être relevées et abaissées séparément ou ensemble dans la direction z et
**en ce que** les récipients de réactif (R) peuvent être placés dans la zone de traitement (12) en y étant insérés sous un angle (α) en correspondance à la grille des éléments de transfert (28) et peuvent être jetés dans un collecteur de déchets e étant expulsés dans la même direction.

2. Dispositif (10) selon la revendication 1, **caractérisé en ce que** les éléments de transfert (28) sont configurés sous la forme d'aimants permanents ou d'électroaimants, de préférence en forme de barreaux.

3. Dispositif selon les revendications 1 ou 2,
**caractérisé en ce que** la partie la plus basse des éléments de transfert (28), celle qui pénètre dans les récipients à échantillons (P) et les récipients à réactifs (R), est recouverte par une membrane (M) configurée de préférence en forme de tube ou de godet, apte à être relevée et abaissée, et placée et retirée par un déplacement relatif par rapport aux éléments de transfert (28).

4. Dispositif (10) selon l'une des revendications 1 à 3, **caractérisé en ce que** l'angle (α) entre la direction x et la direction y est de 90°.

5. Dispositif (10) selon l'une des revendications 1 à 4, **caractérisé en ce que** le déplacement relatif d'un élément de transfert (28) par rapport à la membrane (M) correspondante dans le sens (z) de la longueur de cette membrane s'effectue en relevant ou en abaissant différemment les plaques de support (24b, 24c) et la membrane (M).

6. Dispositif (10) selon l'une des revendications 1 à 5, **caractérisé en ce qu'**un troisième guide qui permet d'amener en continu des membranes (M) est disposé sous un angle (β) de 60 à 120° par rapport à la direction x.

7. Dispositif (10) selon l'une des revendications 1 à 6, **caractérisé en ce qu'**un bloc porteur (46) qui présente pour les récipients à réactif (R) des canaux (48) qui s'étendent perpendiculairement à la direction x est disposé dans la zone de traitement (12), les récipients à réactif présentant chacun dans leurs parois latérales (47) deux rainures horizontales (66) ouvertes frontalement, opposées et situées au même niveau.

8. Dispositif (10) selon la revendication 6, **caractérisé en ce que** des poutres (72) aptes à se déplacer horizontalement en va-et-vient et qui présentent des aimants permanents (74) disposés dans la zone occupée par les éléments de transfert (28) aptes à être abaissés pour la remise en suspension et le mélange des microparticules (76), sont disposées dans des découpes (50) qui s'étendent parallèlement aux canaux (48).

9. Récipient à échantillons, récipient à réactifs (P, R) et membranes (M) destinés à être utilisés dans un dispositif (10) selon l'une des revendications 1 à 8, **caractérisés en ce qu'**ils sont configurés comme cassettes essentiellement en forme de ruban et empilables présentant plusieurs cavités (22, 36) en forme de godet qui correspondent à la grille des éléments de transfert (28) prévus dans l'élément de transfert (24).

10. Récipient à échantillons et récipient à réactifs (P, R) selon la revendication 9, **caractérisés en ce que** les cavités (22), de préférence au nombre de six à dix, ont une section transversale de forme aplatie ou ovale et **en ce que** leurs dimensions intérieures en coupe transversale ne sont de préférence que peu supérieures aux dimensions correspondantes des éléments de transfert (28) ou des membranes (M) placées.

11. Procédé de séparation automatique de la phase solide et de la phase liquide d'une suspension et de nettoyage de la phase solide à l'aide d'un dispositif (10), de récipients à échantillons (P) et de récipients à réactifs (R) selon l'une des revendications 1 à 10,
**caractérisé en ce que**
le déplacement de l'élément de support (24) vers l'avant dans la direction x lorsqu'on utilise des barreaux en aimant permanent comme éléments de transfert (28) dotés de membranes (M) chargées et étirées s'effectue en branchant le courant et le déplacement vers l'arrière dans la direction opposée à la direction x lorsque l'on utilise des barreaux en aimant permanent comme éléments de transfert (28) sans membranes (M) ou lorsque l'on utilise des électroaimants en forme de barreaux s'effectue en débranchant le courant.

12. Procédé selon la revendication 11, **caractérisé en ce que** les récipients à échantillons (P) remplis sont d'abord amenés par intermittence ou en continu dans le sens de leur longueur dans la direction x et les récipients à réactifs (R), remplis différemment ou au plus de manière presque identique sont amenés en continu et frontalement dans la zone de traitement (12) dans la direction y,
**en ce que** lors du lancement de chaque nouveau cycle de travail, une membrane (M) est passée au-dessus des éléments de transfert (28) situés le plus en arrière dans la direction x, les éléments de transfert (28) sont abaissés dans les récipients à échantillons (P) placés dans la zone de traitement (12) et sont relevés hors des liquides de suspension avec la membrane (M) après agglomération des microparticules magnétiques (76) sur la membrane (M),
**en ce que** l'élément de support (24) est déplacé dans la direction x vers l'avant d'une unité de grille qui correspond à la distance (a) entre deux récipients à réactifs (R),
**en ce que** le récipient à échantillons (P) débarrassé des particules est expulsé dans un collecteur à déchets et les récipients à réactifs (R) remplis sont en même temps amenés dans la zone de traitement (12),
**en ce que** l'élément de support (24) est abaissé avec les éléments de transfert (28) dans le récipient à réactifs (R), les éléments de transfert (28) sont sortis des membranes (M), les microparticules magnétiques (76) accumulées sont remises en suspension, la suspension (78) est mélangée et les éléments de transfert (28) sont reculés de la distance (a) dans la direction opposée à la direction x pendant que les membranes (M) conservent leur position.

13. Procédé selon les revendications 11 ou 12,
**caractérisé en ce que** lors de chaque déplacement de l'élément de support (24) dans la direction x, les membranes (M) sont entraînées d'une unité de grille et expulsées dans un collecteur de déchets à l'extrémité de la zone de traitement (12).

14. Procédé selon l'une des revendications 11 à 13, **caractérisé en ce que** le dernier récipient à réactifs (R) expulsé hors de la zone de traitement (12) dans la direction x est amené vers la poursuite de son traitement.

15. procédé selon l'une des revendications 11 à 14, **caractérisé en ce qu'**un cycle de travail dure de 2 à 4 min.

16. Procédé selon l'une des revendications 11 à 15, **caractérisé en ce qu'**il utilise des récipients à réactifs (R) qui présentent différents réactifs, mais avec le même réactif dans toutes les cavités (22) d'un même récipient à réactifs (R).
